# EUROPEAN PATENT APPLICATION

(11) **EP 4 538 700 A1**
(43) Date of publication of application: **16.04.2025**
(21) Application number: 22945668.6
(22) Date of filing: 10.06.2022
(51) Int. Cl.: G01N 31/22, G01N 33/483, G01N 33/58

(54) **KIT AND METHOD FOR SELF-SAMPLING EARWAX AND FOR IN SITU MEASUREMENT OF BIOMARKERS USING SAID EARWAX**

(71) Applicant: HERANE-VIVES, Andrés, SW9 6FJ, London (GB)
(72) Inventor: HERANE-VIVES, Andrés, SW9 6FJ, London (GB)
(74) Representative: Fuchs Patentanwälte Partnerschaft mbB
(86) International application number: PCT/IB2022/055394
(87) International publication number: WO 2023/237914

(57) **Abstract**

The invention relates to a kit and a method for self-sampling earwax and for in situ measurement of biomarkers using said earwax. The kit comprises a device for taking samples and, additionally, a kit for directly measuring biomarkers of earwax present in the sampling kit.

## Description

### BACKGROUND OF THE INVENTION

### A. FIELD OF THE INVENTION

The present invention is related to a method and a kit for taking earwax samples to measure the concentration of various biomarkers, particularly a method and a kit for taking earwax samples and measuring various biomarkers using a portable kit.

### B. DESCRIPTION OF THE STATE OF THE ART

As described in international patent application Publication No. WO2019/123392A1 of the same applicant, earwax or cerumen samples are reliable for reflecting the chronic systemic level of substances highly variable in the short term, such as cortisol and glucose. Likewise, this document describes an efficient and safe device for extracting earwax samples for analysis.

In addition to said sample extraction device, it would be highly desirable to have a device that allows biomarker levels to be measured directly from the sample extraction device *in situ* rather than *in vitro.* The prior art does not describe or suggest any device or method for measuring biomarker levels from earwax.

In view of the above, the applicant developed a kit that includes said device for extracting earwax samples and, additionally, a portable device that can use batteries or electric current as a power source for measuring biomarkers directly from the earwax present in the device for extracting earwax samples.

Said kit basically comprises a device for sample extraction similar to that described in WO2019/123392A1 and a portable device for measuring biomarkers, designed to receive the sponge of the device for sample extraction previously impregnated with the earwax sample. Said device for measuring biomarkers uses a cylindrical multiplexed lateral flow biosensor that has different assays side by side. However, other biosensors, such as those called "amperemeters", may also be used. The proposed device for measuring biomarkers has cameras that collect the images of the tests, which are sent to information processing means so that they can interpret the images received from the tests using an immunochromatographic program.

The information processing means having a light signal detector that includes cameras. Test results are displayed on a liquid crystal display (LCD).

In this way, a user can practically and rapidly obtain the concentration of various biomarkers from a biological sample of earwax taken by the same user.

### SUMMARY OF THE INVENTION

Therefore, the present invention's primary objective is to provide a kit and method for self-sampling earwax and measuring biomarkers *in situ* from the said earwax.

Another main objective of the present invention is to provide a kit and a method of the nature described above, which includes a device for taking samples and additionally a device for measuring biomarkers directly from the earwax present in the sampling kit. Another objective of the present invention is to provide a kit and a method of the nature described above, wherein the device for measuring biomarkers is designed to receive the sponge of the device for sample extraction with the sample of earwax and uses a cylindrical multiplexed lateral flow biosensor that has different tests next to each other, and where the device for measuring markers has cameras that collect the images of the tests which are sent to information processing means to interpret the images received from the tests using an immunochromatography program, but other methods could also be used.

These and other objectives and advantages of the kit and method for self-sampling earwax and for measuring *in situ* biomarkers from said earwax of the present invention would be apparent to those who have ordinary skill in the art from the following detailed description of the embodiments of the invention to be made with reference to the accompanying drawings.

### BRIEF DESCRIPTION OF THE DRAWINGS

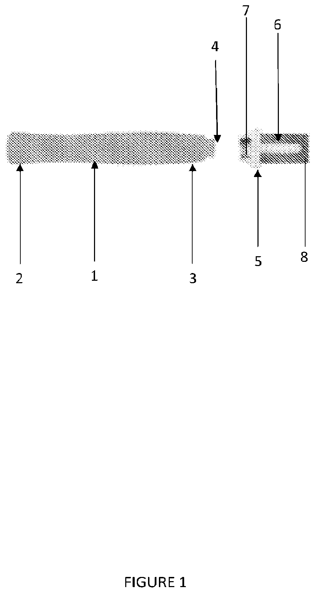

Figure 1 is a left-side view of the earwax removal device of the present invention, showing the cross-section of the tip, including the sponge.

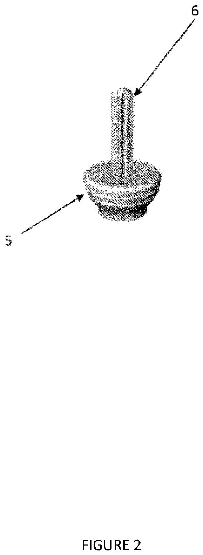

Figure 2 is a perspective view of the tip of the earwax removal device of the present invention without the sponge.

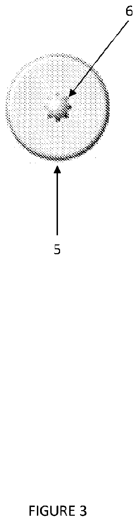

Figure 3 is a top view of the tip of the earwax removal device of the present invention without the sponge.

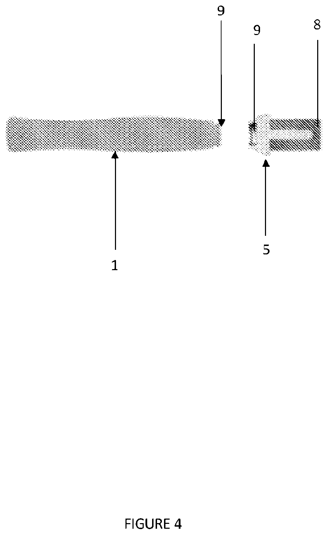

Figure 4 is a left-side view of a second embodiment of the earwax removal device of the present invention, showing the cross-section of the tip, including the sponge.

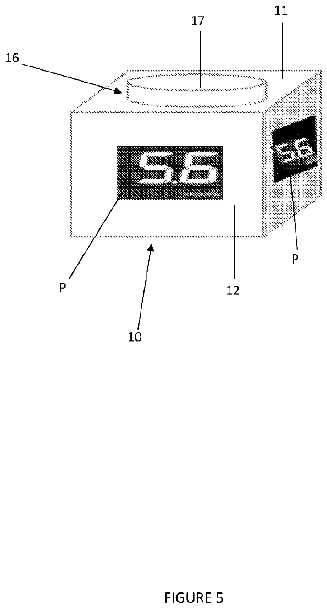

Figure 5 is a perspective view of the device for measuring biomarkers of the present invention.

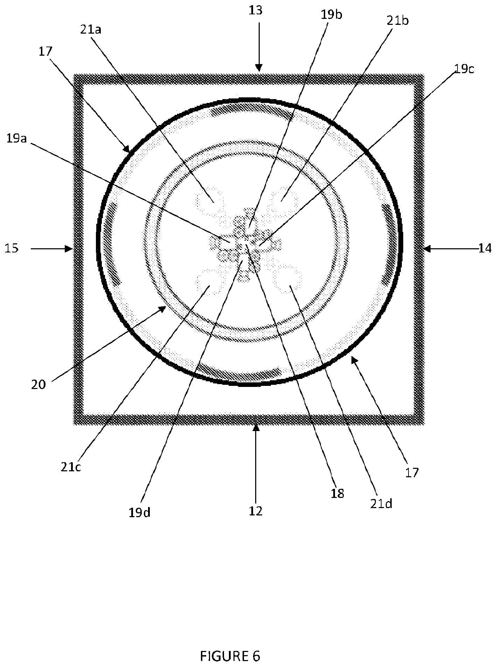

Figure 6 is a top view of the biomarker measurement device of the present invention without the top wall.

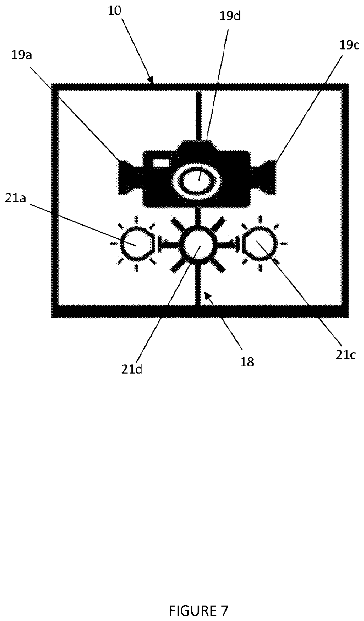

Figure 7 is a front view of the device for measuring biomarkers of the present invention without the front wall, showing its internal components.

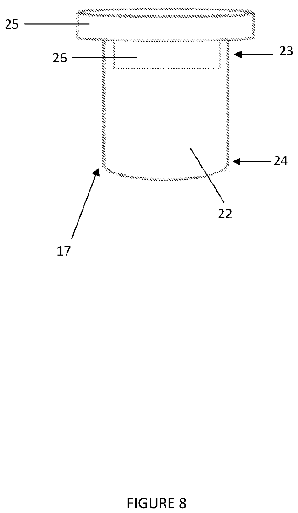

Figure 8 is a front view of the biosensor holder of the present invention.

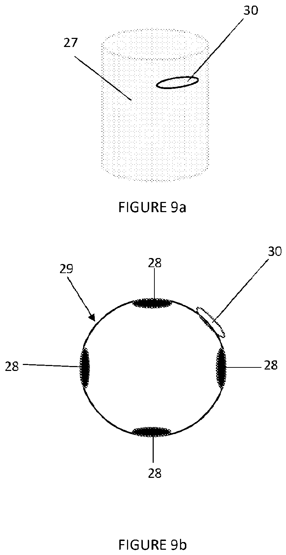

Figure 9a is a perspective view of the biosensor unit of the present invention.
Figure 9b is a top view of the biosensor unit of the present invention.
Figure 10a is a front component diagram of the multiplexed lateral flow biosensor of the present invention.
Figure 10b is a perspective view of the biosensor unit of the present invention without the front portion, showing the location of the multiplexed lateral flow biosensor adhered to the inner surface of the flexible backing plate.
Figures 11 a, 11 b, and 11 c show a front view of the nitrocellulose membrane of the multiplexed lateral flow biosensor of the present invention, showing the test line and the control line in various states.
Figure 12 is a front view of the device for removing earwax of the present invention, showing its sponge inserted in the biosensor unit, which is, in turn, inserted in the biosensor holder.
Figure 13 is a perspective view of the device for measuring biomarkers, with the biosensor holder, including the biosensor unit, about to be inserted into the device for measuring biomarkers through its circular central opening.

### DETAILED DESCRIPTION OF THE INVENTION

The kit for self-sampling of earwax and for the measurement of biomarkers in situ from said earwax will now be described concerning a preferred embodiment thereof, where said kit comprises:
A device for the extraction of samples similar to that described in WO2019/123392A1, the description of which is included below for reference:
A handle (1) that has a first end (2) and a second end (3), said second end (3) having coupling means that In a preferred embodiment of the invention, they may comprise a thread (4);
A detachable head (tip) comprising a base (5) and a longitudinally extendable elongated sponge holder (6) directly depending on an upper part of the base, in which the lower part of the base has a shell that includes a pattern internal threading (7) to receive the thread (4) of the handle (1), and in which the sponge of the support (6) has a star-shaped cross-section;
An elongated sponge (8) having a centrally located longitudinal casing (not shown) having a star-shaped cross-section to receive the sponge holder (6) of the base (5). where the handle (1) and the base (5) can include any coupling means suitable for coupling the handle, such as a pressure joint (9) and
wherein the sponge (8) may preferably be made of cellulose and glued to the sponge holder (6) using a non-allergenic glue.

A device for measuring biomarkers from an earwax sample obtained by means of the sample extraction device, comprising:
a cube-shaped housing (10), having: an upper wall (11), a lower wall (not shown), a front wall (12), a rear wall (13), a right-side wall (14) and a left-side wall (15), where the upper wall (11) has a central circular opening (16) to receive a cylindrical biosensor holder (17);
information processing means (not shown) mounted on the lower wall of the housing (10);
a pedestal (18) anchored to a middle portion of the surface of the lower wall of the housing (10), said pedestal (18) having a height less than the walls of the housing (10);
four cameras (19a, 19b, 19c, 19d) mounted in the upper third of the pedestal (18), each camera (19a, 19b, 19c, 19d) being directed to a middle portion of a right lateral wall (14), left lateral wall (15), corresponding front wall (12) and rear wall (13) and each camera (19a, 19b, 19c, 19d) being connected to the information processing means;
focusing means to allow a better resolution of the image obtained by the cameras; said focusing means comprising a circular lens (20) that surrounds the pedestal (18) that has the cameras (19a, 19b, 19c, 19d) and the cylindrical biosensor holder (17) and held by support means (not shown) anchored to the lower wall;
the light source, wherein a preferred embodiment comprises four light sources (21 a, 21 b, 21 c, 21 d) mounted on the pedestal (18) below the cameras (19a, 19b, 19c, 19d) and each light source is connected to a power source (not shown), where each light source (21 a, 21 b, 21c, 21 d) produces a light with specific monochromatic spectral characteristics in a wave range between 600nm -1400nm to guarantee a high contrast between the background and the specific staining zone given by the reaction in each of the four biosensors, which will be described later;
at least one screen (P) for presenting information, each connected to the information processing means for presenting measurement results;

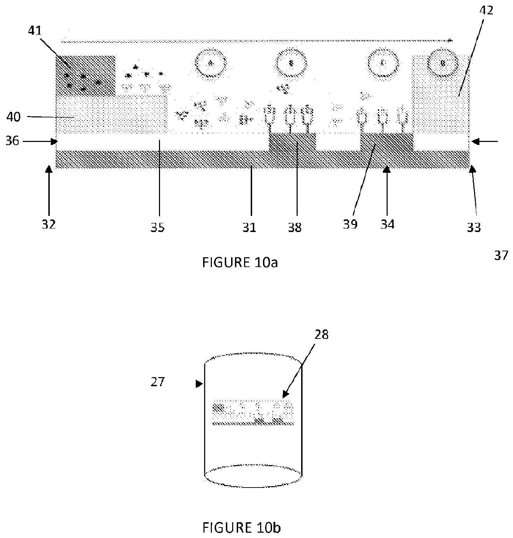

a biosensor holder (17) comprising a hollow cylindrical shaped element having a cylindrical wall with an external surface (22) and an internal surface (not shown), an upper end (23) and a lower end (24), a wall of upper end (25) that has a larger diameter than the cylindrical element, leaving the lower end open, where an upper portion of the cylindrical element has a slot (26) with a length of +/- 4 mm wherein the cylindrical-shaped element has such a diameter that it allows it to pass tightly through the central circular opening (16) of the device for measuring biomarkers;
a biosensor unit (27) of cylindrical shape, which has at least one multiplexed lateral flow biosensor (28), shown in Figure 10, where the biosensor unit (27) comprises:
   a flexible backing plate (29) having a hollow cylindrical shape with open ends made of a plastic material such as vinyl, but other materials can also be used that have a solid binding to proteins (including antibodies or enzymes) and variable absorption properties (different capillary flow times), such as nitrocellulose, said cylindrical backing plate having an external surface and an internal surface and a diameter and height such that it can be inserted tightly into the biosensor holder (17), and said backing plate having stop means (30) on an upper portion of the external surface that engages in the slot (26) of the biosensor holder (17) when the backing plate (29) is inserted into the biosensor holder (17);
   one or more multiplexed lateral flow biosensors (28), each positioned equidistantly on a middle portion of the inner surface of the flexible backing plate (29) following the diameter of the backing plate, each comprising:
      A base membrane (31), elongated in shape, made of a flexible material selected from the set that includes but is not limited to vinyl or nitrocellulose, said base membrane being sensitive to pressure through the use of an adhesive made of acrylic but, non-reactive and stable. Said base membrane (31) having a first end (32), a second end (33), an internal surface and an external surface (34), where the internal surface may have hydrophilic characteristics to slow down the diffusion of the sample of wax (hydrophobic), thus guaranteeing the time necessary for the immunohistochemical reaction to take place;
      a nitrocellulose membrane (35) of elongated shape, having a first end (36) and a second end (37) and having a test line portion (38) and a control line portion (39), said nitrocellulose membrane (35) located vertically on the base membrane (31) along its entire length where its first end (36) and second end (37) coincide with the first end (32) and second end (33) of the base membrane (31);
      a conjugate pad (40) located vertically on the nitrocellulose membrane at a first horizontal end (36) thereof to contain conjugated tracer antibodies;
      An earwax sample pad (41) located vertically on the conjugate pad (40). The sample pad (41) is made of a material with high oil absorption properties, such as natural inorganic substances like sand, clay or volcanic ash. It can also be made of other natural organic and synthetic materials and must be pre-treated with a buffer solution;
   an absorbent pad (42) located in a vertical direction on the nitrocellulose membrane (35) at a second horizontal end (37) thereof;
The components of each biosensor (28) are attached to the internal surface of the backing plate (29) of the biosensor unit (27) using an adhesive substance. In turn, each component of each biosensor (28) is attached to the base membrane (31) in close contact with each other in such a way that the migration of sample and test components through the biosensor (28) is ensured. (also called a test strip) during a test, and preferably overlap the end where it joins another component by approximately 2 mm.

Earwax (cerumen) diffuses into the sample pad (41), which will act as a filter to facilitate the flow of sample and test components. The conjugates to be used (enzymes or antibodies) are dispensed with a pipette onto the conjugate pad (40). In other embodiments, the pads may be pre-prepared separately, dried at room temperature, and stored in deaccectors at 4 °C before use and attachment to the biosensor. The corresponding capture reagents are dispensed onto the membrane. Nitrocellulose (35), where the test lines (38) and control lines (39) are located respectively by any device or suitable method.

The labels (tracers) are placed on the pads and nitrocellulose membrane and are preferably made of colloidal gold, carbon, or latex. Specific tags are chosen based on their compatibility with the test components.

Once the specific analytes in the earwax have reached the conjugate pad (40), they will rehydrate conjugated tracer antibodies to match each other (zone (A) shown in Figure 10).

The resulting complexes move through the principle of capillarity from the area marked (A) to the area marked (D) until they are detected on the test line (38) via a previously deposited detection antibody (zone (B)). In case there is no junction on the test line (38), a negative result is displayed.

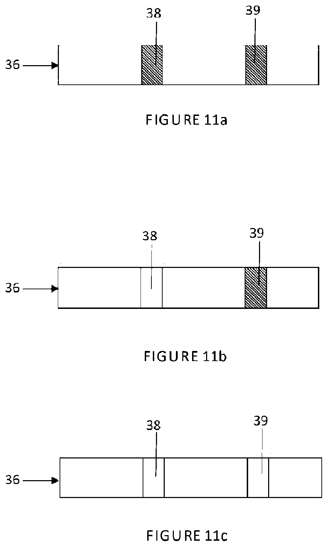

The potential results (staining) provided by each biosensor (28) are shown In Figure 11a showing a positive result, 11b shows a negative result, and 11c shows an invalid result.

In the test line (38), the gold-labeled antibody recognizes a unique part of the target molecule, known as an epitope, and binding occurs.

Unreacted antibody/tracer conjugates are finally detected in the control line (39) by a species-specific antibody (zone (C)). Both lines, test (38) and control (39), contain capture reagents to display the results.

The visual control line (39) indicates that the test has been executed correctly. The labels colour the control (39) and test (38) lines.

Ultimately, the presence of an absorbent pad (42) will collect any excess samples and reagents (zone (D)).

To perform a test, the cylindrical biosensor unit (27) that has one or more (preferably up to four multiplexed lateral flow biosensors (28) previously prepared and with the earwax sample is inserted into the biosensor holder. (17), which in turn is inserted into the central circular opening (16) of the device for measuring biomarkers in such a way that the upper-end wall (25) of the biosensor holder (17) serves as a stop with the upper wall (11) of the device for measuring biomarkers. When the biosensor holder (17) is inserted into the device for measuring biomarkers, the internal surface of the backing plate (29) of the biosensor unit (27) surrounds the pedestal (18) with the cameras (19a, 19b, 19c, 19d), in such a way that each chamber (19a, 19b, 19c, 19d) is pointing at the test lines (38) and control (39), also called test strip or staining zone of a lateral flow biosensor multiplexed (28);

Each camera (19a, 19b, 19c, 19d) obtains images of the test line (38) and the control line (39) (staining zone) as data from a matrix of points arranged in horizontal and vertical rows. Each point, or pixel, is characterized by three numerical parameters, corresponding to three image formation channels - Red, Green and Blue (RGB) - that comprise the RGB circuit. The recording results for each channel are digits that increase as the staining intensity increases.

The images obtained by each camera (19a, 19b, 19c, 19d) are processed in information processing means by means of Immunochromatography software, which tests four readings which will correspond to the chronic level of the substances measured in the earwax. The results are shown on the screens (P), where each one corresponds to a camera (19a, 19b, 19c, 19d) and its corresponding biosensor (28). In other embodiments, the software responsible for processing the information from the cameras (19a, 19b, 19c, 19d) may comprise an Artificial Intelligence algorithm that can be trained to improve the interpretation of the images obtained and, therefore, the measurement of the biomarkers.

### Use of the kit

Previously, the user must prepare the multiplexed lateral flow biosensors (28) as follows:
- Apply the conjugates to be used (enzymes or antibodies) on the conjugate pad (40).
- Apply the corresponding capture reagents on the nitrocellulose membrane (35), where the test lines (38) and control lines (39) are located, respectively.

Insert the biosensor unit (27) cylindrically into the biosensor holder (17) so that the stop means (30) of the backing plate (29) hooks into the slot (26) of the biosensor holder, securing the biosensor unit (27) inside the biosensor holder (17).

The sample extraction is preferably carried out with a sample extraction device similar to that described in WO2019/123392A1, whose description was included above as a reference. However, the extraction can be carried out with any other type of device as long as it has a sponge that can adapt to the shape and size of the cylindrical opening of the biosensor unit (27) inserted in the biosensor holder (17) and that has sufficient length to contact the sample pad (41) of each of the biosensors (28) with an adequate amount of sample. In a preferred embodiment, it is possible to analyze cortisol and glucose from all previously obtained earwax samples with a sample as small as 0.8 mg of earwax, so the minimum sample amount is preferably 0.8 mg.

Earwax is obtained by inserting the sponge tip (8) of the sampling device into the ear canal and rotating the sponge inside the ear canal for approximately 30 to 60 seconds.

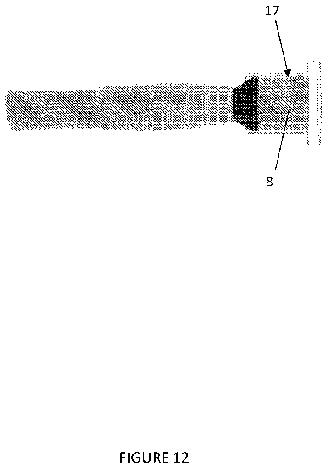

Once the earwax sample is obtained, the user must insert the sponge tip of the sample extraction device into the cylindrical biosensor unit located inside the biosensor holder, as shown in the figure. Figure 12.

Inside the biosensor unit (27), the user must rotate the sponge (8) in such a way that the sponge (8) rubs the sample pad (41) of each biosensor (28), depositing a biological wax sample to ears (minimum preferably 0.8 mg) in each sample pad (41).

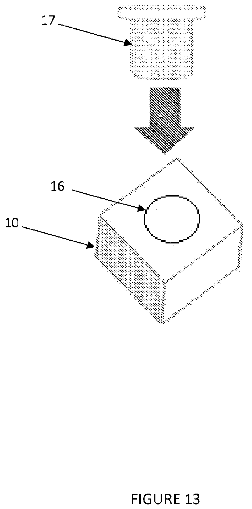

Once the sample pads (41) are impregnated with an earwax sample, the biosensor unit (27) must be inserted into the central circular opening (16) of the device for measuring biomarkers so that the upper-end wall (25) of the biosensor holder serves as a stop with the upper wall (11) of the device for measuring biomarkers, as shown in Figure 13.

To ensure that each visual control line (38), (39) is aligned with a respective camera (19a, 19b, 19c, 19d), the biosensor holder (17) may include guidance means (not shown) that cooperate with the circular central opening (16) of the housing.

As described previously, the device for measuring biomarkers uses the translation of different electrochemical signals as a reading mechanism for different biomarkers, such as the glucose present in human earwax. However, other optical reading mechanisms could also be used.

The degree of fluorescence determines a specific electrical signal and concentration of the analyte to be determined, such as glucose, through specific calibration curves stored in the device's memory. Other conjugates, such as gold or silver nanoparticles that emit different wavelengths that can also be translated into different concentrations, may also be used.

In other embodiments, the device for measuring biomarkers of the present invention can use different types of biosensors, such as, for example, but not limited to amperometric, potentiometric, impedimetric, voltammetric, piezoelectric, thermometric, optical, etc. biosensors.

Some of the said biosensors do not require cameras since the measurement results can be provided through signals sent to the information processing means, either through a direct connection or wirelessly. So, in said modalities, cameras are not required. It would not be necessary to use the pedestal with the cameras, lighting, or focusing means. In the same way, it is possible that the biosensors used do not require prior preparation by the user (apply the conjugates to be used and apply the corresponding capture reagents).

Likewise, in other embodiments, the sponge (8) of the device for sample extraction, the central opening (16) of the device for measuring biomarkers, and the cross-section of the biosensor holder (17) and the biosensor unit (27) can have any cross-sectional shape, as long as it allows the sponge (8) to deposit the appropriate amount of earwax on the biosensor. Furthermore, the biosensor holder (17) may only comprise a structure and means for holding one or more sensors.

In the same way, the housing (10) of the device for measuring biomarkers can have any cross-sectional shape, such as circular, or even only comprise a structure with means to hold the biosensor holder (17).

Finally, in other embodiments, the biosensor holder (17) may not be used, and only the biosensor unit (27) may be inserted into the device for measuring biomarkers. The unit must have a cavity so that the sponge of the sampling device can be inserted to impregnate the sensors with the earwax sample.

It should finally be understood that the kit and method for self-sampling of earwax and for measuring in situ biomarkers from said earwax of the present invention is not limited to the modality described above and that those skilled in the art will be qualified, by the teachings established here, to make changes in the kit and method for self-sampling of earwax and for the measurement of biomarkers in situ from said earwax of the present invention, whose scope will be established exclusively by the following claims.

## Claims

1. A device for measuring biomarkers from an earwax sample obtained by a sample extraction device, comprising a handle, a head attached to the handle, said head having a sponge holder and a sponge attached to the holder for a sponge for sampling earwax by inserting it into the ear canal, wherein said device for measuring biomarkers comprises:
a casing having at least one upper wall wherein the upper wall has a central opening;
information processing means mounted in an internal portion of the housing;
at least one screen for presenting information, each connected to the information processing means for presenting results of biomarker measurements;
one or more biosensors, each placed within the housing, are contactable by the sponge of the sampling device so that said sponge can deposit a sample of earwax in each biosensor.

2. A device for measuring biomarkers according to claim 1, additionally having:
a lower wall:
a camera holder pedestal anchored to a middle portion of the surface of the lower wall of the housing;
at least one camera mounted on an upper portion of the pedestal, each camera being positioned such that it is directed to a respective biosensor and each camera being connected to the information processing means;
focusing means surrounding the pedestal and one or more cameras to allow better resolution of the image obtained by the cameras;
a light source located inside the housing.

3. A device for measuring biomarkers according to claim 2, wherein the focusing means comprises a circular lens surrounding the pedestal that has the cameras and the cylindrical biosensor and is held by support means anchored to the lower wall; and where said light source produces light illumination with monochromatic spectral characteristics in a wave range between 600nm and 1400nm.

4. A device for measuring biomarkers, according to claim 1, wherein one or more biosensors are attached to a removable biosensor unit, comprising:
a flexible backing plate of hollow cylindrical shape with open ends made of a material plastic that has a strong binding to proteins, including antibodies or enzymes and variable absorption properties (different capillary flow times), such as nitrocellulose, said backing plate of cylindrical shape having an external surface and an internal surface, wherein Each biosensor is attached to the inner surface of the backing plate so;
where the biosensor unit is inserted into the device to measure biomarkers through the central opening.

5. A device for measuring biomarkers according to claim 1, wherein the central opening of the housing has a cylindrical shape and wherein the device for measuring biomarkers additionally comprises:
a biosensor holder comprising a hollow cylindrical shaped element that has a cylindrical wall with an external surface and an internal surface, an upper end and a lower end, an upper-end wall that has a greater diameter than the cylindrical-shaped element, the lower end being open, where the cylindrical-shaped element It has such a diameter that it allows it to fit snugly through the circular central opening of the device for measuring biomarkers;
one or more biosensors are attached to a biosensor unit, which comprises a flexible backing plate of hollow cylindrical shape with open ends made of a plastic material that has a strong binding to proteins, including antibodies or enzymes, such as nitrocellulose; said cylindrical backing plate having an external surface and an inner surface and diameter and height such that it can be inserted snugly into the biosensor holder, where each biosensor is attached to the inner surface of the backing plate so and where the biosensor unit is inserted into the biosensor holder. The biosensor holder is inserted into the central opening of the housing, which has a cylindrical shape.

6. A device for measuring biomarkers according to claim 1, wherein the central opening of the housing has a cylindrical shape and wherein the device for measuring biomarkers additionally comprises:
a lower wall:
a camera holder pedestal anchored to a middle portion of the bottom wall surface of the casing;
at least one camera mounted on an upper portion of the pedestal, each camera being positioned such that it is directed to a respective biosensor and each camera being connected to the information processing means;
focusing means surrounding the pedestal and one or more cameras to allow better resolution of the image obtained by the cameras;
a light source located inside the housing.
a biosensor holder comprising a hollow cylindrical shaped element having a cylindrical wall with an external surface and an internal surface, an upper end and a lower end, an upper-end wall having a greater diameter than the cylindrically shaped element, being the open lower end, where the cylindrical-shaped element has a diameter such that it allows it to pass tightly through the circular-shaped central opening of the device for measuring biomarkers;
one or more biosensors are attached to a biosensor unit, which comprises a flexible backing plate of hollow cylindrical shape with open ends made of a plastic material that has a strong binding to proteins, including antibodies or enzymes, such as nitrocellulose; said backing plate of cylindrical shape having an external surface and an internal surface and a diameter and a height such that can be inserted snugly into the biosensor holder, where each biosensor is attached to the inner surface of the backing plate so and where the biosensor unit is inserted into the biosensor holder and the biosensor holder is inserted inside the central opening of the casing that has a cylindrical shape;
and where:
each biosensor has a reactive strip (staining zone);
the upper-end wall of the biosensor holder serves as an abutment with the upper wall of the device for measuring biomarkers;
When the biosensor holder is inserted in the device for measuring biomarkers, the internal surface of the backing plate of the biosensor unit surrounds the pedestal with the cameras in such a way that each camera is pointing to a test strip (area staining) of a multiplexed lateral flow biosensor;
the images obtained by each camera are processed in information processing media through Immunochromatography software, which tests four readings corresponding to the chronic level of the substances measured in the earwax. The results are shown on the screens, where each corresponds to a camera and its corresponding biosensor.

7. A device for measuring biomarkers according to claim 6, wherein each sensor is a multiplexed lateral flow biosensor comprising:
a base membrane of an elongated shape, made of a flexible material selected from the set comprising but not limited to vinyl or nitrocellulose, said the base membrane is pressure sensitive through the use of an adhesive made of acrylic but non-reactive and stable. Said base membrane has a first end, a second end, an internal surface and an external surface, where the internal surface may have hydrophilic characteristics, to slow down the diffusion of the wax sample (hydrophobic), thus guaranteeing the time necessary for it to the immunohistochemical reaction is carried out;
a nitrocellulose membrane, elongated in shape, having a first end and a second end and having a portion of the test line and a portion of the control line, said nitrocellulose membrane located vertically on the base membrane along its entire length where its first and second ends coincide with the first and second ends of the base membrane;
a conjugate pad located vertically on the nitrocellulose membrane at the first horizontal end thereof to contain conjugated tracer antibodies;
An earwax sample pad is placed vertically above the conjugate pad. The sample pad is made of high oil absorption property material, such as natural inorganic substances like sand, clay, or volcanic ash. It can also be made of other natural organic and synthetic materials and must be pre-treated with a buffer solution;
and an absorbent pad located vertically on the nitrocellulose membrane at a second horizontal end thereof;
where: the earwax diffuses into the sample pad, which also acts as a filter;
the conjugates to be used (enzymes or antibodies) are dispensed onto the conjugate pad;
the corresponding capture reagents are dispensed onto the nitrocellulose membrane where the test and control lines are located, respectively;
labels (tracers) located on the pads and nitrocellulose membrane are included and are made of a material selected but not limited to colloidal gold, carbon or latex;
The resulting complexes during the assay move by the principle of capillarity until they are detected on the test line via a pre-deposited detection antibody.

8. A kit for self-sampling of earwax and for measuring biomarkers in situ from said earwax comprising:
A device for sample extraction, comprising a handle, a head attached to the handle, said head having a sponge holder and a sponge attached to the sponge holder for sampling earwax by inserting it into the ear canal;
The device for measuring biomarkers from an earwax sample obtained by a sample extraction device of claims 1 to 7.

9. A method for self-sampling of earwax and for measuring biomarkers in situ from said earwax comprising:
a. extracting a sample of earwax using a device for removing earwax comprising a handle, a head attached to the handle, said head having a sponge holder and a sponge attached to the sponge holder for taking a sample of earwax by inserting it into the ear canal;
b. depositing earwax using the sponge of the device for removing earwax in the one or more biosensors of the device to measure biomarkers of claims 1 to 7.

10. A multiplexed lateral flow biosensor comprising:
a base membrane, elongated, made of a flexible material selected from the set comprising but not limited to vinyl or nitrocellulose, said base membrane is pressure sensitive through an adhesive made of acrylic but non-reactive and stable. Said base membrane having a first end, a second end, an internal surface and an external surface, where the internal surface may have hydrophilic characteristics, to slow down the diffusion of the earwax sample (hydrophobic), thus guaranteeing the time necessary for the immunohistochemical reaction to be carried out;
a nitrocellulose membrane of elongated shape, having a first end and a second end and having a test line portion and a control line portion, said nitrocellulose membrane located vertically on the base membrane along the entire length of the same where its first and second end coincides with the first and second end of the base membrane;
a conjugate pad located vertically on the nitrocellulose membrane at the first horizontal end thereof to contain conjugated tracer antibodies;
an earwax sample pad is placed vertically above the conjugate pad. The sample pad is made of high oil absorption property material, such as natural inorganic substances like sand, clay, or volcanic ash. It can also be made of other natural organic and synthetic materials and must be pre-treated with a buffer solution;
and an absorbent pad located vertically on the nitrocellulose membrane at a second horizontal end thereof;
where:
the earwax diffuses into the sample pad, which also acts as a filter;
the conjugates to be used (enzymes or antibodies) are dispensed onto the conjugate pad;
the corresponding capture reagents are dispensed onto the nitrocellulose membrane where the test and control lines are located, respectively;
labels (tracers) located on the pads and nitrocellulose membrane are included and are made of a material selected but not limited to colloidal gold, carbon or latex;
and the resulting complexes during the assay move through the principle of capillarity until they are detected on the test line through a pre-deposited detection antibody.
